# EUROPÄISCHE PATENTANMELDUNG

(11) **EP 0 635 849 A1**
(43) Veröffentlichungstag der Anmeldung: **25.01.1995**
(21) Anmeldenummer: 93110111.7
(22) Anmeldetag: 24.06.1993
(51) Int. Cl.: G21K 1/093, A61N 5/10

(54) **Umlenkung eines sich entlang einer Achse auf einen Zielpunkt zubewegenden Teilchenstrahls**

(71) Anmelder: SIEMENS AKTIENGESELLSCHAFT, D-80333 München (DE)
(72) Erfinder: Farley, Francis, Prof., F-06620 Le Bar-sur-Loup (FR)

(57) **Zusammenfassung**

Die Erfindung betrifft ein Verfahren zur Umlenkung eines sich entlang einer Achse (1) auf einen Zielpunkt (2) zubewegenden Teilchenstrahls (3) auf eine die Achse (1) in dem Zielpunkt (2) kreuzende Zielgerade (4). Hierbei wird der Teilchenstrahl (3) vor dem Zielpunkt (2) auf eine erste Zwischengerade (5) umgelenkt, welche mit einer von der Achse (1) und der Zielgeraden (4) aufgespannten Hauptebene (6) einen von Null verschiedenen Winkel bildet. Erfindungsgemäß wird der Teilchenstrahl (3) von der ersten Zwischengeraden (5) auf eine zweite Zwischengerade (7), welche die erste Zwischengerade (5) mit der Zielgeraden (4) verbindet, und von der zweiten Zwischengeraden (7) auf die Zielgerade (4) in den Zielpunkt (2) umgelenkt. Die Erfindung betrifft auch eine Vorrichtung zur Durchführung eines solchen Verfahrens. Die Erfindung findet vor allem Anwendung in Führungssystemen für Teilchenstrahlen (3), insbesondere für Teilchenstrahlen (3) von Protonen mit Energien von einigen hundert MeV, welche im Rahmen der Therapie, insbesondere der Tumortherapie, eingesetzt werden.

## Beschreibung

Die Erfindung betrifft ein Verfahren zur Umlenkung eines sich entlang einer Achse auf einen Zielpunkt zubewegenden Teilchenstrahls auf eine die Achse in dem Zielpunkt kreuzende Zielgerade, wobei der Teilchenstrahl vor dem Zielpunkt auf eine erste Zwischengerade umgelenkt wird, welche mit einer von der Achse und der Zielgeraden aufgespannten Hauptebene einen von Null verschiedenen Winkel bildet.

Derartige Verfahren finden Anwendung in Beschleunigeranlagen für geladene Teilchen wie Protonen oder Elektronen, die der Therapie, insbesondere der Tumor-Therapie, dienen. Sie sind insbesondere wichtig in Führungssystemen, die es erlauben, Teilchenstrahlen aus einer Vielzahl vorgebbarer Richtungen auf einen Patienten zu richten. Solche Führungssysteme, die mehr oder weniger aufwendige Magnetanordnungen enthalten, sind in der Regel um eine Achse, entlang derer der Teilchenstrahl eintritt, schwenkbar und gestatten die Zustellung des Teilchenstrahls zu einem gewünschten Zielpunkt aus einem Winkelbereich bis 360°.

Verfahren zur Umlenkung eines sich entlang einer Achse auf einen Zielpunkt zubewegenden Teilchenstrahls gehen hervor aus dem US-Patent 4,812,658 und dem US-Patent 4,870,287. Das Führungssystem für den Teilchenstrahl, das jeweils zur Durchführung des Verfahrens dient und schwenkbar ist, um eine variable Zustellung des Teilchenstrahls zum jeweiligen Patienten zu ermöglichen, wird jeweils als "gantry" bezeichnet. Die "gantry" wird jeweils aus einer Teilchenbeschleunigeranlage mit einem Teilchenstrahl beaufschlagt.

Gemäß dem US-Patent 4,870,287 erfolgt in dem Führungssystem die Umlenkung des entlang einer Achse, um die das Führungssystem schwenkbar ist, zugestellten Teilchenstrahls im wesentlichen in einer einzigen Ebene, in der der Teilchenstrahl dreimal um einen Winkel von jeweils 90° umgelenkt wird. Nach der dritten Umlenkung befindet sich der Strahl auf einer Zielgeraden senkrecht zur Achse, wobei er sich auf die Achse zubewegt. Der Patient, bzw. der zu behandelnde Bereich des Patienten, ist üblicherweise in die Nähe des Schnittpunktes der Zielgeraden mit der Achse, welcher als Zielpunkt anzusehen ist, zu bringen. Das Führungssystem beansprucht einen relativ großen Raum; um eine Schwenkung um 360° zu ermöglichen, ist ein zylindrischer Freiraum von beachtlicher Größe erforderlich.

Das US-Patent 4,812,658 beschreibt ein Verfahren zur Umlenkung eines sich entlang einer Achse auf einen Zielpunkt zubewegenden Teilchenstrahls auf eine die Achse in dem Zielpunkt kreuzende Zielgerade, wobei der Teilchenstrahl vor dem Zielpunkt auf eine erste Zwischengerade umgelenkt wird, welche mit einer von der Achse und der Zielgeraden aufgespannten Hauptebene einen von Null verschiedenen Winkel bildet. Gleichfalls beschrieben ist eine "gantry" zur Durchführung des Verfahrens. Der Teilchenstrahl wird von der Achse zunächst um einen Winkel von 45° abgelenkt und mittels einer weiteren Ablenkung um 45° in eine zur Achse senkrechte Ebene gebracht, in der die Zielgerade mit dem Zielpunkt liegt. Nach der zweifachen Ablenkung bewegt sich der Strahl in dieser Ebene, allerdings nicht zum Zielpunkt hin, sondern von dem Zielpunkt fort. Durch in der Ebene erfolgende Umlenkungen um insgesamt 270°, ausgeführt mittels zweier Umlenkungen um je 135°, wird der Strahl auf die Zielgerade gebracht und auf den Zielpunkt gerichtet. Dieses Verfahren erfordert insbesondere dann, wenn eine Schwenkung der für die Ausübung vorgesehenen "gantry" um 360° möglich sein soll, einen geringeren Platzbedarf als das aus dem US-Patent 4,870,287 ersichtliche Verfahren; allerdings ist der Platzbedarf in der Ebene, in der sich die Zielgerade befindet, außerordentlich hoch. Dies liegt daran, daß in dieser Ebene zwei verschiedene Magnetanordnungen in radialer Richtung hintereinander angeordnet werden müssen, nämlich eine Magnetanordnung für eine Ablenkung um 45°, die den Teilchenstrahl in die Ebene hineinbringt, sowie eine Magnetanordnung zur Bewirkung einer Ablenkung um 135°, um den Teilchenstrahl letztendlich zum Zielpunkt zu bringen. Neben einem kegeligen Freiraum für den von der Achse um 45° abgelenkten Teilchenstrahl wird daher ein relativ großer Freiraum in der Ebene, die die Zielgerade enthält, benötigt.

Zur Auslegung von Magnetanordnungen im allgemeinen sei, außer auf die bereits genannten Patente, auf das US-Patent 4,442,352 verwiesen. Dieses Patent zeigt eine Magnetanordnung, die nicht nur einen Teilchenstrahl ablenkt, sondern zusätzlich mit Hilfe des Teilchenstrahls Abbildungen nach Art der Optik durchführt. "Strahlenoptik" ist in den meisten Magnetanordnungen zur Ablenkung und Führung von Teilchenstrahlen von großer Bedeutung, um die Teilchenstrahlen zu fokussieren, das heißt zu verhindern, daß sie sich unkontrolliert aufweiten und somit verloren gehen. Insoweit ist jede Magnetanordnung zu analysieren als strahlenoptisches System dahingehend, ob ein von dieser Magnetanordnung geführter Teilchenstrahl als solcher erhalten bleibt oder durch gewisse Instabilitäten oder dergleichen zerstört wird. Ein wichtiges strahlenoptisches Kriterium für die Güte einer Magnetanordnung ist ihre Chromatizität, welche Korrelationen zwischen dem Ort und dem Impuls eines Teilchens in dem Teilchenstrahl oder zwischen der Inklination der Bahn des Teilchens relativ zu einer Achse des Teilchenstrahls sowie dem Impuls des Teilchens angibt. Weist eine Magnetanordnung Chromatizität auf, so bedeutet dies, daß die Bahn, die ein Teilchenstrahl durchfliegt, mehr oder weniger stark von der Energie der Teilchen dieses Teilchenstrahls abhängt, und daß auch der Querschnitt des Teilchenstrahls in Abhängigkeit von der Energie beeinflußt werden kann. Es ist daher stets wünschenswert, Magnetanordnungen ohne Chromatizität, das heißt achromatische Magnetanordnungen, zu erzielen. Solche Magnetanordnungen sind hinsichtlich ihrer Eigenschaften zur Führung eines Teilchenstrahls stabil dahingehend, daß sie von gewissen Schwankungen der Energie der Teilchen weitestgehend unbeeinflußt bleiben. Ein Dipolmagnet, welcher zur Winkelablenkung eines Teilchenstrahls dient, hat stets eine gewisse Chromatizität; diese Chromatizität kann jedoch unter Umständen ausgeglichen werden dadurch, daß der eine Dipolmagnet aufgeteilt wird in zwei Dipolmagnete, welche hintereinander liegen und insgesamt dieselbe Winkelablenkung wie der ursprüngliche einzige Dipolmagnet bewirken. Eine eventuell verbleibende Chromatizität kann durch das Einfügen von Quadrupolmagneten zwischen die beiden Dipolmagnete auf alle Fälle restlos beseitigt werden.

In Ansehung des Standes der Technik beruht die Erfindung auf der Aufgabe, ein Verfahren zur Umlenkung eines sich entlang einer Achse auf einen Zielpunkt zubewegenden Teilchenstrahls der in der Einleitung genannten Art anzugeben, welches mit geringem Aufwand an Raum und Material realisierbar ist und welches vorzugsweise keine Probleme bezüglich einer Chromatizität mit sich bringen soll. Darüber hinaus soll im Rahmen der Erfindung eine Vorrichtung zur Durchführung dieses Verfahrens angegeben werden.

Zur Lösung dieser Aufgabe ist das erfindungsgemäße Verfahren zur Umlenkung eines sich entlang einer Achse auf einen Zielpunkt zubewegenden Teilchenstrahls auf eine die Achse in dem Zielpunkt kreuzende Zielgerade, wobei der Teilchenstrahl vor dem Zielpunkt auf eine erste Zwischengerade umgelenkt wird, welche mit einer von der Achse und der Zielgeraden aufgespannen Hauptebene einen von Null verschiedenen Winkel bildet, dadurch gekennzeichnet, daß der Teilchenstrahl von der ersten Zwischengeraden auf eine zweite Zwischengerade, welche die erste Zwischengerade mit der Zielgeraden verbindet, und von der zweiten Zwischengeraden auf die Zielgerade in den Zielpunkt umgelenkt wird.

Ein wesentlicher Unterschied des erfindungsgemäßen Verfahrens zu dem aus dem US-Patent 4,812,658 hervorgehenden Verfahren liegt darin, daß anstelle der vorbekannten Umlenkung eines Strahls in zwei Ebenen, nämlich einer ersten Ebene, die die Achse enthält, und einer zweiten Ebene, die senkrecht zur Achse ist, ersetzt wird durch ein Verfahren, welches eine Umlenkung des Teilchenstrahls in drei verschiedenen und winklig zueinander angeordneten Ebenen beinhaltet. Die erste Ebene ist dabei eine Ebene, die die Achse und die erste Zwischengerade enthält, die zweite Ebene wird aufgespannt von der ersten Zwischengerade und der zweiten Zwischengerade, und die dritte Ebene enthält die zweite Zwischengerade und die Zielgerade. Zu bemerken ist, daß keine dieser Ebenen senkrecht zur Achse steht. Unbeschadet der möglichen Verwendung zweiteiliger Magnete für die im Rahmen des Verfahrens notwendigen Umlenkungen ist es in keinem Fall erforderlich, daß im Bereich der Zielgeraden mehrere Magnetanordnungen für Umlenkungen hintereinander angebracht werden müssen; daher wird radial um den Zielpunkt herum eine wesentliche räumliche Ersparnis erreicht. Im Gegensatz zu dem US-Patent 4,812,658 sind auch nur drei Magnetanordnungen anstelle von vieren erforderlich; dementsprechend ist auch der apparative Aufwand erfindungsgemäß beachtlich verringert. Da die Erfindung mit sich bringt, daß zweimal die Ebenen, in denen der Teilchenstrahl abgelenkt wird, gewechselt werden, wird eine "gantry" zur Durchführung des Verfahrens auch als "supertwist-gantry" bezeichnet.

Das erfindungsgemäße Verfahren ist auch, trotz der in der Tat dreidimensionalen Struktur des zur Ausübung notwendigen Führungssystems - im Hinblick auf die zu wünschende Achromatizität - unproblematisch, da es in der Tat möglich ist, alle auftretenden Chromatizitäten mit einfachen Mitteln zu kompensieren und somit zu einer vollständig achromatischen Struktur zu kommen.

Vorteilhafterweise wird das Verfahren in der Weise realisiert, daß die Zielgerade etwa senkrecht zu der Achse ausgerichtet ist. Auf diese Weise kann eine relativ einfache geometrische Struktur erhalten werden, wozu noch näheres auszuführen ist, und der Platzbedarf wird besonders gering gehalten. Auch ist eine Ausrichtung der Zielgeraden senkrecht zur Achse von besonderer Wichtigkeit im Zusammenhang mit Anwendungen des Verfahrens zu therapeutischen Zwecken, beispielsweise zur Behandlung von Tumoren. Ein wesentlicher Vorteil der Behandlung von Tumoren mit Protonen oder anderen relativ schweren Teilchen, welche auf kinetische Energien von einigen hundert MeV beschleunigt sind, liegt darin, daß die in einen Körper eintretenden Teilchen zunächst nur wenig mit dem Gewebe reagieren und schließlich ihre Energie in einem relativ eng begrenzten räumlichen Gebiet an das Gewebe abgeben. Somit ist es möglich, eine Tiefenbehandlung von Tumoren, die unter gesunden Gewebeschichten liegen, durchzuführen. Um dennoch die Schädigung gesunden Gewebes gering zu halten, ist es vorteilhaft, den Weg, den die Teilchen durch das gesunde Gewebe nehmen müssen, so gering wie möglich zu halten. Hierfür ist im Rahmen des Verfahrens die Wahl einer zur Achse senkrechten Zielgeraden im Regelfall besonders günstig.

Ebenfalls besonders vorteilhaft ist es, die erste Zwischengerade etwa senkrecht zu der Achse auszurichten und damit den Teilchenstrahl zu Beginn um etwa 90° abzulenken. Für eine derartige Ablenkung stehen Realisierungsmöglichkeiten für eine entsprechende Umlenkmagnetanordnung in großer Zahl zur Verfügung, was den Entwicklungsaufwand wesentlich verringert. Auch ergibt sich eine besonders einfache geometrische Struktur, und darüber hinaus kann die Umlenkmagnetanordnung hinsichtlich ihrer Chromatizität so ausgelegt werden, daß sie eine verbleibende Chromatizität der übrigen Umlenkmagnetanordnungen, die zur Durchführung des Verfahrens erforderlich sind, kompensiert. Derart ist also die Schaffung eines vollständig achromatischen Führungssystems möglich. Von besonderem Vorteil ist es, die erste Zwischengerade senkrecht zur Hauptebene, also senkrecht zu der von der Achse und der Zielgeraden aufgespannten Ebene, auszurichten. Diese Wahl ist insbesondere im Hinblick auf die Vermeidung von Chromatizität von besonderer Bedeutung.

Besonders vorteilhaft ist es auch, wenn die Konfiguration aus der Achse, der ersten Zwischengeraden und der zweiten Zwischengeraden derart gewählt wird, daß eine auf der ersten Zwischengeraden von der Achse und der zweiten Zwischengeraden begrenzte Strecke etwa gleich lang ist wie eine auf der Zielgeraden von der Achse und der zweiten Zwischengeraden begrenzten Strecke. Diese Weiterbildung ist im Hinblick auf ihre Symmetrie besonders vorteilhaft; dies insbesondere deshalb, weil die Wahl symmetrischer Umlenkmagnetanordnungen in der Regel die Vermeidung von Chromatizität unterstützt.

Im Rahmen einer besonders vorteilhaften Weiterbildung, die vorzugsweise zu der soeben beschriebenen Weiterbildung hinzutritt und bei der besonders vorzugsweise die Zielgerade etwa senkrecht zur Achse und die erste Zwischengerade etwa senkrecht zur Hauptebene ausgerichtet ist, ist eine auf der Achse von der ersten Zwischengeraden und der Zielgeraden begrenzte Strecke kleiner als die auf der Zielgeraden liegende Strecke. Diese Ausgestaltung ermöglicht den Einsatz eines entlang der Achse besonders kompakten Führungssystems mit allen sich daraus ergebenden Vorteilen. Darüber hinaus ist festzuhalten, daß die Hinzunahme aller vorzugsweise erwähnten Merkmale dazu führt, daß die für den Teilchenstrahl zwischen der ersten Zwischengerade und der zweiten Zwischengerade bzw. der zweiten Zwischengeraden und der Zielgeraden zu realisierenden Ablenkwinkel etwa gleich, und zwar geringfügig Kleiner als, 135° sind. Für derartige Ablenkwinkel stehen erprobte Umlenkmagnetanordnungen zur Verfügung. Darüber hinaus vermag eine verbliebene Chromatizität einer Anordnung aus zwei zueinander verdrehten Umlenkmagnetanordnungen für 135° kompensiert zu werden mit einer entsprechend ertüchtigten Umlenkmagnetanordnung zwischen der Achse und der ersten Zwischengeraden, wo der Teilchenstrahl um etwa 90° abgelenkt wird. Mit besonderem Vorteil ist außerdem die Strecke auf der Zielgeraden zumindest dreimal so lang wie die Strecke auf der Achse; in einem solchen Fall kann ein besonders kompaktes Führungssystem verwendet werden, und die Winkel, um die die Umlenkmagnetanordnungen zwischen den Zwischengeraden bzw. der zweiten Zwischengeraden und der Zielgeraden umlenken müssen, weichen allenfalls um wenige Grad von 135° ab. Demgemäß sind ohne weiteres übliche und erprobte Umlenkmagnetanordnungen einsetzbar.

Zur Lösung der Aufgabe der Erfindung wird auch eine Vorrichtung zur Durchführung des erfindungsgemäßen Verfahrens angegeben, welche eine erste Umlenkmagnetanordnung zur Umlenkung des Teilchenstrahls von der Achse auf die erste Zwischengerade, eine zweite Umlenkmagnetanordnung zur Umlenkung des Teilchenstrahls von der ersten Zwischengeraden auf die zweite Zwischengerade sowie eine dritte Umlenkmagnetanordnung zur Umlenkung des Teilchenstrahls von der Zwischengeraden auf die Zielgerade und auf den Zielpunkt aufweist. Jede Umlenkmagnetanordnung kann aus einem einzigen Umlenkmagneten oder mehreren einander zugeordneten Umlenkmagneten bestehen. Hierzu sei darauf hingewiesen, daß eine Zuordnung mehrerer Umlenkmagnete zueinander im Rahmen einer Umlenkmagnetanordnung in erster Linie dadurch gegeben ist, daß die Umlenkmagnete in ein und derselben Ebene ablenken; auf die räumliche Nähe der Umlenkmagnete zueinander kommt es nicht unbedingt an. Es ist auch nicht auszuschließen, daß zwischen einander zugeordneten Umlenkmagneten Steuermagnete nach Art eines Quadrupolmagnets oder dergleichen angeordnet sind. Steuermagnete sind unter anderem dadurch gekennzeichnet, daß sie keine Ablenkung eines Teilchenstrahls hervorrufen, sondern allenfalls seine Form beeinflussen. Dementsprechend beeinträchtigen Steuermagnete nicht die Zusammengehörigkeit mehrerer Umlenkmagnete in einer Umlenkmagnetanordnung, welche gegeben ist dadurch, daß die Umlenkmagnete den Teilchenstrahl in ein und derselben Ebene ablenken. Die erfindungsgemäße Vorrichtung führt die bereits erwähnte Umlenkung des Teilchenstrahls in drei verschiedenen Ebenen durch und entspricht damit insbesondere dann, wenn sie im Rahmen einer Einrichtung zur Therapie mit Teilchenstrahlen realisiert ist, der bereits erwähnten "supertwist-gantry".

Die erste Umlenkmagnetanordnung bewirkt vorzugsweise eine Umlenkung des Teilchenstrahls um etwa 90° und kann damit in bekannter Weise realisiert werden.

Die zweite Umlenkmagnetanordnung und die dritte Umlenkmagnetanordnung sind mit besonderem Vorteil in der Weise symmetrisch ausgeführt, insbesondere dadurch, daß sie den Teilchenstrahl um jeweils etwa gleiche Winkel ablenken. Dieses Merkmal ist insbesondere im Hinblick auf die Problematik der Chromatizität von besonderer Bedeutung. Die erwähnten Winkel liegen dabei vorzugsweise zwischen 125° und 135°, wodurch die Vorrichtung besonders kompakt realisierbar ist.

Die Vorrichtung ist unbeschadet zusätzlicher Weiterbildungen vorteilhafterweise derart ausgelegt, daß der Teilchenstrahl durch die dritte Umlenkmagnetanordnung senkrecht zur Achse umgelenkt wird, da auch dieses Merkmal sowohl zu einer besonders einfachen und symmetrischen Geometrie als auch zu einem verringerten Platzbedarf beiträgt.

In die Vorrichtung jedweder anderweitigen Ausgestaltung sind vorteilhafterweise außer Umlenkmagneten, welche die Umlenkmagnetanordnungen bilden, Steuermagnete nach Art von Quadrupolmagneten und dergleichen einbezogen, was sowohl zur Erzielung einer im ganzen achromatischen Vorrichtung beiträgt als auch eine feine Justierung des Teilchenstrahls in der Vorrichtung erlaubt. Letzteres ist von besonderer Bedeutung im Zusammenhang mit therapeutischen Anwendungen, da ein Teilchenstrahl mit Teilchen, insbesondere Protonen, mit einer Energie im Bereich einiger hundert MeV immerhin eine beachtliche Wirkung auf lebendes Gewebe ausübt und daher schon aus Gründen des Strahlenschutzes und der Vermeidung unnötiger Belastungen eine jederzeit reproduzierbare Position des Teilchenstrahls im Bereich des gewünschten Zielpunktes notwendig ist. Es sei bemerkt, daß es bei einem Steuermagnet, insbesondere einem Quadrupolmagnet, nicht nur auf die entsprechende Positionierung entlang der Bahn des Teilchenstrahls ankommt, sondern auch auf die richtige Einstellung seiner vielen, in einer Ebene senkrecht zum Teilchenstrahl um diesen herum verteilten Magnetpole. Für einen Quadrupolmagneten, der einer Umlenkmagnetanordnung zugeordnet ist, gibt es in diesem Sinne zwei besondere Einstellungen relativ zu der Ebene, in der die Umlenkmagnetanordnung eine Ablenkung bewirkt. Im Rahmen einer ersten Einstellung befinden sich von den vier Magnetpolen des Quadrupolmagneten zwei über und zwei unter der Ebene; in einer zweiten Einstellung liegen zwei Magnetpole in der Ebene und zwei Magnetpole über und unter der Ebene. Es sei bemerkt, daß die Ausmerzung einer Chromatizität unter Umständen mehrere Quadrupolmagnete erfordern kann, mit denen beide möglichen Einstellungen realisiert werden müssen.

Mit besonderem Vorteil ist die Vorrichtung jedweder Ausgestaltung vollständig achromatisch, was heißt, daß sie den Teilchenstrahl in einer zumindest in einem gewissen Bereich der Energie seiner Teilchen von der Energie unabhängigen Weise führt, unter der Voraussetzung, daß die Eigenschaften des Teilchenstrahls beim Eintritt in die Vorrichtung unabhängig von der Energie sind.

Für medizinische Anwendungen ist die Vorrichtung beispielsweise ausgelegt für Teilchenstrahlen von Protonen, wobei die Energie jedes Protons zwischen etwa 10 MeV und 500 MeV, vorzugsweise zwischen 100 MeV und 300 MeV, liegt. Es versteht sich, daß eine Vorrichtung nicht jedes der genannten Intervalle der Energie vollständig abdecken muß, sondern unter Umständen nur für ein entsprechend kleines Teilintervall ausgelegt sein kann entsprechend der gewünschten Anwendung.

Die Vorrichtung jedweder Ausgestaltung ist mit besonderem Vorteil drehbar um die Achse und erlaubt somit, den Teilchenstrahl dem Zielpunkt aus einem mehr oder weniger großen Winkelbereich zuzustellen. Gerade dieses Merkmal qualifiziert die Vorrichtung in besonderer Weise für die Anwendung im Rahmen der Therapie mittels Teilchenstrahlen.

Ausführungsbeispiele der Erfindung sind aus der Zeichnung ersichtlich. Im einzelnen zeigen:
Figur 1 eine Skizze in dimetrischer Projektion, welche ein Beispiel für die der Erfindung zugrundeliegende Geometrie zeigt;
Figur 2 eine Schrägansicht eines Führungssystems zur Führung eines Teilchenstrahls gemäß der Erfindung;
Figur 3 eine Seitenansicht des aus Figur 2 ersichtlichen Führungssystems.

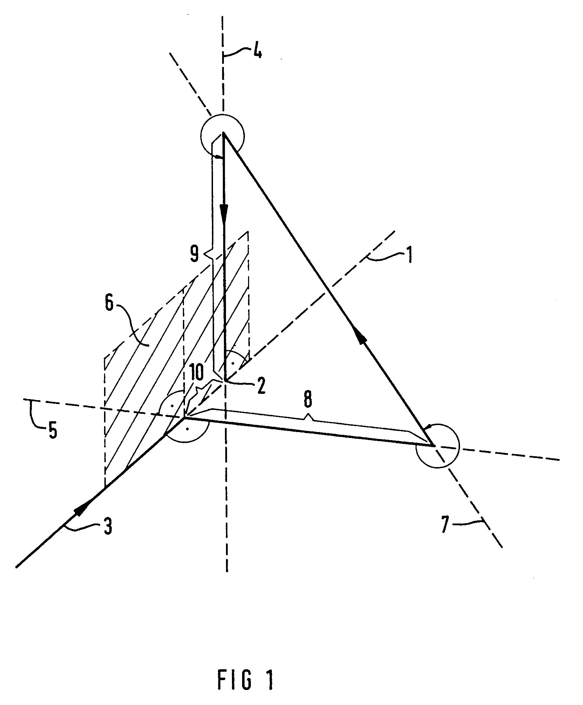

In Figur 1 sind die Achse 1, die erste Zwischengerade 5, die zweite Zwischengerade 7 und die Zielgerade 4 als gestrichelte Linien dargestellt; die Bahn des Teilchenstrahls 3 ist durch eine ausgezogene Linie angedeutet. Der Einfachheit halber ist diese Bahn jeweils durch die Schnittpunkte der verschiedenen Geraden 1, 4, 5 und 7 fortgesetzt, was selbstverständlich nicht dem Regelfall bei der Realisierung einer solchen Bahn entspricht. Dies tut allerdings der prinzipiellen Bedeutung der Figur 1 keinen Abbruch. Der Teilchenstrahl 3 bewegt sich zunächst entlang der Achse 1 hin zu dem Zielpunkt 2. Vor dem Zielpunkt 2 wird er abgelenkt auf die erste Zwischengerade 5. Von der ersten Zwischengeraden 5 gelangt der Teilchenstrahl 3 auf die zweite Zwischengerade 7, die, abseits der Achse 1 im Raum liegend, die auf der Achse 1 liegende Strecke 10 zwischen der ersten Zwischengeraden 5 und dem Zielpunkt 2 überbrückt. Von der zweiten Zwischengeraden 7 wird der Teilchenstrahl 3 schließlich entlang der Zielgeraden 4 in den Zielpunkt 2 gelenkt. In diesem Zielpunkt 2 ist beispielsweise ein mit dem Teilchenstrahl 3 zu behandelndes Gewebe anzuordnen; der Übersicht halber ist weder dieses Gewebe noch die weitere Bahn des Teilchenstrahls 3 dargestellt. Von Bedeutung ist, daß die erste Zwischengerade 5 mit der Hauptebene 6, die von der Achse 1 und der Zielgeraden 4 aufgespannt wird und von der ein schraffierter Ausschnitt ersichtlich ist, einen von Null verschiedenen Winkel bildet - im aus Figur 1 ersichtlichen Beispiel ist die Zwischengerade 5 senkrecht zur Hauptebene 6 ausgerichtet. Die Zielgerade 4 schneidet die Achse 1 in einem rechten Winkel. Weiterhin sind die Strecke 8 auf der ersten Zwischengeraden 5 zwischen der Achse 1 und der zweiten Zwischengeraden 7 sowie die Strecke 9 auf der Zielgeraden 4 zwischen der zweiten Zwischengeraden 7 und der Achse 1 gleich lang. Auf diese Weise wird die Bahn des Teilchenstrahls 3 in besonderer Weise symmetrisch; insbesondere ist der Winkel zwischen der Zwischengeraden 5 und der zweiten Zwischengeraden 7 gleich dem Winkel zwischen der zweiten Zwischengeraden 7 und der Zielgeraden 4; beide Winkel betragen darüber hinaus etwa 135°, und dies um so exakter, je größer die Strecken 8 und 9 relativ zur Strecke 10 sind. Solange die Strecken 8 und 9 länger sind als die Strecke 10, liegen die erwähnten Winkel zwischen 125° und 135°; ist das Verhältnis zwischen der Strecke 8 (bzw. der gleich langen Strecke 9) und der Strecke 10 wie dargestellt größer als 3, so weichen die Winkel nur wenige Grad von 135° ab, und zwar nach unten. Diese Abweichung ist aber immerhin gering genug, um beispielsweise die Verwendung an sich bekannter Umlenkmagnetanordnungen für einen Winkel von 135° zu erlauben.

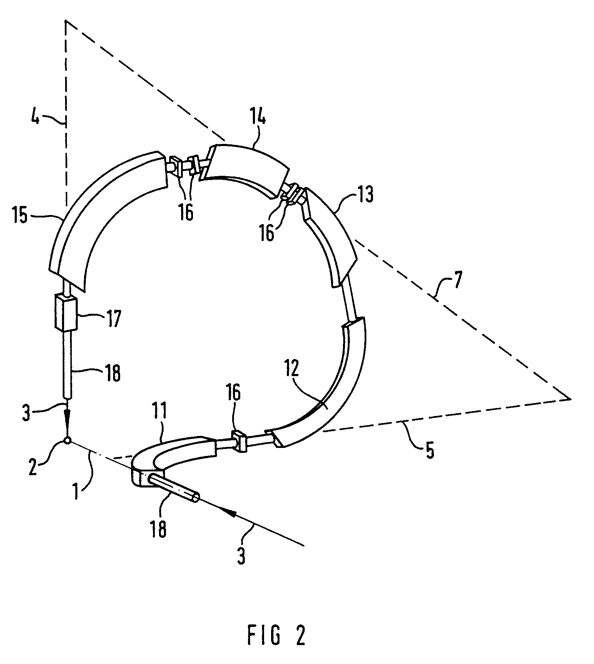

Aus Figur 2 ist, ebenfalls in einer Schrägansicht, ein Ausführungsbeispiel für ein Führungssystem für eine Strahlführung der in Figur 1 skizzierten Art ersichtlich. Auch sind die Achse 1, die erste Zwischengerade 5, die zweite Zwischengerade 7 und die Zielgerade 4 als gestrichelte Linien angedeutet. Auch ist erkennbar ein Strahlrohr 18, welches evakuiert ist und zur Führung des Teilchenstrahls 3 im Vakuum dient. Die Arbeitsweise des Führungssystems ist folgende: Eine erste Umlenkmagnetanordnung 11, im dargestellten Fall ein einzelner Dipolmagnet, lenkt den Teilchenstrahl 3 um 90° ab auf die erste Zwischengerade 5. Eine zweite Umlenkmagnetanordnung 12, 13, die aus zwei voneinander getrennten Umlenkmagneten 12 und 13 besteht, lenkt den Teilchenstrahl 3 auf die zweite Zwischengerade 7, und eine dritte Umlenkmagnetanordnung 14, 15, ebenfalls bestehend aus zwei einander zugeordneten Umlenkmagneten 14 und 15, bringt den Teilchenstrahl 3 auf die Zielgerade 4 und zum Zielpunkt 2, der auf der Achse 1 liegt. Das Führungssystem enthält auch zwischen den Umlenkmagneten 11 und 12, 13 und 14 bzw. 14 und 15 angeordnete Steuermagnete 16, beispielsweise einzelne oder gepaarte Quadrupolmagnete 16, sowie hinter dem Umlenkmagneten 15 einen weiteren Steuermagneten 17. Alle Steuermagnete 16 und 17 dienen der Justierung der Lage des Teilchenstrahls 3 in dem Führungssystem sowie der Eliminierung von Chromatizitäten, die durch die Umlenkmagnete 11, 12, 13, 14 und 15 bedingt sein können. Es sei bemerkt, daß der einzelne Umlenkmagnet 11 bereits ausreichen kann, eine aufgrund der Tatsache, daß die zweite Umlenkmagnetanordnung 12, 13 und die dritte Umlenkmagnetanordnung 14, 15 in voneinander verschiedenen Ebenen ablenken, verbliebene Chromatizität zu kompensieren. Dennoch empfiehlt es sich in der Regel, für Zwecke der Fokussierung und/oder Justierung der Lage des Teilchenstrahls 3 Steuermagnete 16, 17 vorzusehen.

Aus Figur 2 wird auch deutlich, daß die Zuordnung von Umlenkmagneten 12, 13, 14, 15 zu Umlenkmagnetanordnungen nicht unbedingt etwas zu tun hat mit der räumlichen Nähe der einander zugeordneten Umlenkmagnete 12 und 13 bzw. 14 und 15; die Zuordnung erfolgt nach den Ebenen, in denen die einzelnen Umlenkmagnete 12, 13, 14, 15 den Teilchenstrahl 3 umlenken. Dieser Sachverhalt ist bereits vorstehend erläutert worden.

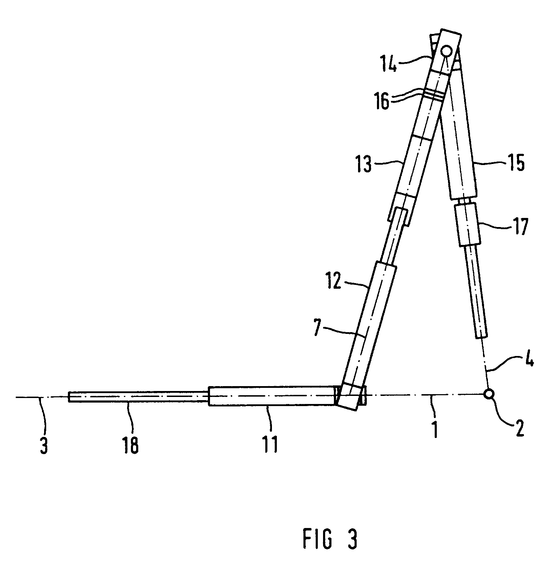

Figur 3 zeigt eine Seitenansicht des Führungssystems nach Figur 2, gesehen entlang der ersten Zwischengeraden 5. Zur Erläuterung der Figur 3 ist daher zunächst auf die entsprechenden Ausführungen zu Figur 2 Bezug zu nehmen. Im einzelnen ist zu vermerken, daß der Teilchenstrahl 3, der in einem Strahlrohr 18 geführt ist, zunächst von der ersten Umlenkmagnetanordnung mit dem Umlenkmagneten 11 von der Achse 1 auf die erste Zwischengerade 5 geleitet wird, das heißt aus der Zeichenebene heraus; anschließend bringt ihn die mit den Umlenkmagneten 12 und 13 gebildete zweite Umlenkmagnetanordnung 12, 13 auf die zweite Zwischengerade 7, und die dritte Umlenkmagnetanordnung 14, 15 mit dem Umlenkmagneten 14 und 15 bringt ihn auf die Zielgerade 4, die im dargestellten Beispiel nicht ganz senkrecht zur Achse 1 steht, und in den Zielpunkt 2. Gleichfalls dargestellt ist der Steuermagnet 17; auch sind zwei Steuermagnete 16 zwischen den Umlenkmagneten 13 und 14 angedeutet.

Erfindungsgemäß wird ein Verfahren zur Umlenkung eines sich entlang einer Achse auf einen Zielpunkt zubewegenden Teilchenstrahls auf eine die Achse in dem Zielpunkt kreuzende Zielgerade sowie eine zur Ausübung dieses Verfahrens bestimmte Vorrichtung angegeben, womit ein erheblicher Gewinn sowohl hinsichtlich des benötigten Platzes als auch hinsichtlich der Handhabbarkeit im Hinblick auf Chromatizitäten und dergleichen ergibt. Das Verfahren und die Vorrichtung sind besonders geeignet zum Einsatz im Zusammenhang mit der Tumortherapie mittels Teilchenstrahlen, insbesondere mittels Teilchenstrahlen von Protonen mit Energien im Bereich einiger hundert MeV.

## Patentansprüche

1. Verfahren zur Umlenkung eines sich entlang einer Achse (1) auf einen Zielpunkt (2) zubewegenden Teilchenstrahls (3) auf eine die Achse (1) in dem Zielpunkt (2) kreuzende Zielgerade (4), wobei der Teilchenstrahl (3) vor dem Zielpunkt (2) auf eine erste Zwischengerade (5) umgelenkt wird, welche mit einer von der Achse (1) und der Zielgeraden (4) aufgespannten Hauptebene (6) einen von Null verschiedenen Winkel bildet, **dadurch gekennzeichnet,** daß der Teilchenstrahl (3) von der ersten Zwischengeraden (5) auf eine zweite Zwischengerade (7), welche die erste Zwischengerade (5) mit der Zielgeraden (4) verbindet, und von der zweiten Zwischengeraden (7) auf die Zielgerade (4) in den Zielpunkt (2) umgelenkt wird.

2. Verfahren nach Anspruch 1, bei dem die Zielgerade (4) etwa senkrecht zu der Achse (1) ausgerichtet ist.

3. Verfahren nach Anspruch 1 oder 2, bei dem die erste Zwischengerade (5) etwa senkrecht zu der Achse (1) ausgerichtet ist.

4. Verfahren nach Anspruch 3, bei dem die erste Zwischengerade (5) etwa senkrecht zu der Hauptebene (6) ausgerichtet ist.

5. Verfahren nach einem der Ansprüche 1 bis 4, bei dem eine auf der ersten Zwischengeraden (5) von der Achse (1) und der zweiten Zwischengeraden (7) begrenzte Strecke (8) etwa gleich lang ist wie eine auf der Zielgeraden (4) von der Achse (1) und der zweiten Zwischengeraden (7) begrenzte Strecke (9).

6. Verfahren nach Anspruch 5, bei dem eine auf der Achse (1) von der ersten Zwischengeraden (5) und der Zielgeraden (4) begrenzte Strecke (10) kleiner ist als die auf der Zielgeraden (4) liegende Strecke (9).

7. Verfahren nach Anspruch 6, bei dem die Strecke (9) auf der Zielgeraden (4) zumindest dreimal so lang wie die Strecke auf der Achse (1) ist.

8. Verfahren nach einem der vorhergehenden Ansprüche, welches achromatisch ist.

9. Vorrichtung zur Durchführung des Verfahrens nach einem der Ansprüche 1 bis 8, welche eine erste Umlenkmagnetanordnung (11) zur Umlenkung des Teilchenstrahls (3) von der Achse (1) auf die erste Zwischengerade (5), eine zweite Umlenkmagnetanordnung (12, 13) zur Umlenkung des Teilchenstrahls (3) von der ersten Zwischengeraden (5) auf die zweite Zwischengerade (7) sowie eine dritte Umlenkmagnetanordnung (14, 15) zur Umlenkung des Teilchenstrahls (3) von der Zwischengeraden (7) auf die Zielgerade (4) und auf den Zielpunkt (2) aufweist.

10. Vorrichtung nach Anspruch 9, bei der die erste Umlenkmagnetanordnung (11) den Teilchenstrahl (3) um etwa 90° ablenkt.

11. Vorrichtung nach einem der Ansprüche 9 oder 10, bei der die zweite Umlenkmagnetanordnung (12, 13) und die dritte Umlenkmagnetanordnung (14, 15) den Teilchenstrahl (3) um jeweils etwa gleiche Winkel ablenken.

12. Vorrichtung nach Anspruch 11, bei der die zweite Umlenkmagnetanordnung (12, 13) dem Teilchenstrahl (3) um einen Winkel zwischen 125° und 135° ablenkt.

13. Vorrichtung nach einem der Ansprüche 9 bis 12, bei der der Teilchenstrahl (3) durch die dritte Umlenkmagnetanordnung (14, 15) etwa senkrecht zur Achse (1) umgelenkt wird.

14. Vorrichtung nach einem der Ansprüche 9 bis 13, welche außer Umlenkmagneten (12, 13, 14, 15) Steuermagnete (16, 17), insbesondere Quadrupolmagnete (16), aufweist.

15. Vorrichtung nach einem der Ansprüche 9 bis 14, bei der die Anordnung aller Umlenkmagnete (12, 13, 14, 15) und Steuermagnete (16, 17) im wesentlichen vollständig achromatisch ist.

16. Vorrichtung nach einem der Ansprüche 9 bis 15, welche um die Achse (1) drehbar ist.
